(19)

Europäisches
Patentamt
European
Patent Office
Office européen
des brevets

(11) **EP 4 282 334 A1**

(12) **EUROPEAN PATENT APPLICATION**
published in accordance with Art. 153(4) EPC

(43) Date of publication:
**29.11.2023   Bulletin 2023/48**

(21) Application number: **21877922.1**

(22) Date of filing: **01.10.2021**

(51) International Patent Classification (IPC):
**A61B 5/361** (2021.01)        **A61B 5/363** (2021.01)
**A61B 5/00** (2006.01)

(52) Cooperative Patent Classification (CPC):
**A61B 5/00; A61B 5/361; A61B 5/363**

(86) International application number:
**PCT/KR2021/013536**

(87) International publication number:
**WO 2022/075670 (14.04.2022 Gazette 2022/15)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **06.10.2020   KR 20200128485**
**06.10.2020   KR 20200128983**

(71) Applicant: **Sky Labs Inc.**
**Gyeonggi-do 13486 (KR)**

(72) Inventors:
• **LEE, Byung Hwan**
**Yongin-si Gyeonggi-do 16981 (KR)**
• **BAIK, Chang Hyun**
**Suwon-si Gyeonggi-do 16505 (KR)**

• **KIM, Chang Hyun**
**Seoul 05372 (KR)**
• **CHOI, Chang Woo**
**Uiwang-si Gyeonggi-do 16000 (KR)**
• **CHANG, Hyung Min**
**Yongin-si Gyeonggi-do 16843 (KR)**
• **JO, Sung Mi**
**Seoul 08706 (KR)**
• **KIM, Hae Na**
**Seongnam-si Gyeonggi-do 13466 (KR)**
• **KIM, Dae Sung**
**Yongin-si Gyeonggi-do 16928 (KR)**
• **JI, Yu Mi**
**Seongnam-si Gyeonggi-do 13607 (KR)**

(74) Representative: **Giuliano, Natalia**
**C/o Studio Rubino SRL**
**Via Lucrezia della Valle, 84**
**88100 Catanzaro (CZ) (IT)**

(54) **METHOD AND DEVICE FOR ANALYZING ABNORMAL PHYSIOLOGICAL STATE**

(57)    The present invention relates to a device and a method for analyzing an abnormal physiological signal, and the method for analyzing an abnormal physiological signal comprises the steps of: removing bio-signal noise generated by a sensor; estimating the signal quality of the bio-signal for a preset unit time; performing classification of an atrial fibrillation (AF), which is an abnormal signal, on the bio-signal for the unit time to classify same into an AF, which is an abnormal signal, and a normal sinus rhythm (NSR), which is a normal signal; determining whether an AF episode, which is an abnormal state episode, occurs and a duration time thereof, on the basis of the signal quality of preset number of consecutive unit times and the result of the classification of the AF, which is an abnormal signal; and calculating the load of the AF, which is an abnormal signal, on the basis of whether an AF episode, which is an abnormal state episode, occurs and the duration time thereof.

[FIG. 1]

EP 4 282 334 A1

**Description**

Technical Field

The present invention relates to a method of analyzing an abnormal physiological condition such as atrial fibrillation and a device therefor. More particularly, the present invention relates to a method of determining an episode of an abnormal physiological condition such as atrial fibrillation by analyzing a biosignal obtained from a sensor interfaced with a subject and a device therefor.

Background Art

A condition in which the heart beats too slowly, too fast, or irregularly, i.e., an abnormal heartbeat, is called an arrhythmia. Atrial fibrillation (AF) is a type of arrhythmia, in which each atrium does not beat normally and tremors occur irregularly. As a symptom of atrial fibrillation, a rapid and irregular heartbeat is observed.

Atrial fibrillation is a relatively frequent arrhythmia. Atrial fibrillation is the most common symptom among patients with arrhythmia requiring treatment. About 33 % of all arrhythmia-related inpatients are patients with atrial fibrillation. Early and accurate prediction of frequently atrial fibrillation is of great medical significance.

Atrial fibrillation, in which the atria fail to contract normally and the atria tremble slightly, may cause symptoms such as difficulty in breathing and chest pain. In addition to these symptoms, there is an increased risk of more serious and dangerous arrhythmia. Atrial fibrillation prevents blood from being effectively pumped out of the heart, resulting in a variety of serious problems.

Compared to normal people, patients with atrial fibrillation have a 5 -fold increased risk of stroke and a 2-fold increased mortality rate. In addition, due to various heart disease-related complications caused by atrial fibrillation, the mortality rate of patients with atrial fibrillation is about twice that of normal people.

When a pulse rate increases rapidly due to sudden atrial fibrillation, cardiac output (the amount of blood pumped out when the heart contracts) rapidly decreases because there is not enough time to fill the heart with blood. In a normal heart, contraction of the atria accounts for about 30 % of the cardiac output, so a heart rate continuously increases to compensate for the insufficient cardiac output.

As a result of atrial fibrillation, a burden is applied to the heart, resulting in deterioration of the function of the heart and structural changes in the heart. As a result, heart failure may develop or worsen. In addition, when the heart is unable to contract normally due to atrial fibrillation, blood is congested in the heart and the risk of blood coagulation in the heart increases.

These blood clots that form within the heart travel through arteries and block blood vessels in the brain and other parts of the body. Accordingly, the risk of stroke or thromboembolism in patients with atrial fibrillation greatly increases.

When atrial fibrillation is accurately predicted and various atria pacing methods are considered, the possibility of preventing dangerous symptoms such as stroke or thromboembolism may be increased, thereby reducing hospitalization costs and alleviating patient pain.

Therefore, to prevent these various dangerous diseases, a method of accurately determining and analyzing various abnormal physiological conditions in addition to atrial fibrillation described as an example by analyzing various biosignals is required.

Disclosure

Technical Problem

Therefore, the present invention has been made in view of the above problems, and it is one object of the present invention to provide a method of accurately estimating an abnormal condition, such as atrial fibrillation burden, by analyzing a biosignal obtained from a sensor interfaced with a subject and a device therefor.

Technical Solution

In accordance with one aspect of the present invention, provided is a method of analyzing abnormal physiological conditions, including:
a step of removing noise from a biosignal generated by a sensor; a step of estimating signal quality of the biosignal of a preset unit time; a step of classifying the biosignal of a preset unit time as an abnormal signal or a normal signal; a step of determining whether an abnormal condition episode has occurred and a duration time based on signal quality and abnormal signal classification results for biosignals of a plurality of unit times; and a step of calculating an abnormal condition burden based on whether the abnormal condition episode has occurred and the duration time.

In one embodiment, the method includes a step of removing noise from a biosignal generated by a sensor; a step of estimating signal quality of the biosignal of a preset unit time; a step of classifying the biosignal of a preset unit time as atrial fibrillation (AF) or normal sinus rhythm (NSR); a step of determining whether an AF episode has occurred and a duration time based on signal quality and atrial fibrillation classification results for biosignals of a plurality of unit times; and a step of calculating an atrial fibrillation burden based on whether the AF episode has occurred and the duration time.

In one embodiment, the step of calculating an atrial fibrillation burden includes a step of calculating an analysis time by adding duration times of analyzable unit signals among actually collected biosignals; a step of calculating a sum of duration times of atrial fibrillation episodes occurring at the analysis time; and a step of calculating a ratio of the sum of duration times of the atrial fibrillation episodes to the analysis time.

In one embodiment, the analyzable unit signals include a unit signal included in a minimum unit in which signal quality of each of biosignals of a preset minimum number of consecutive unit times is determined to be good; a unit signal included in a minimum unit in which signal quality of at least one of biosignals of a preset minimum number of consecutive unit times is determined to be good and the biosignal is determined as normal sinus rhythm; and all unit signals until it is determined that an AF episode has ended, after each of biosignals of consecutive unit times of more than a preset minimum number is classified as atrial fibrillation and signal quality is determined to be good, after an AF episode occurs, when each of biosignals of a preset minimum number of consecutive unit times is determined as non-atrial fibrillation.

In one embodiment, the step of determining whether an AF episode has occurred and a duration time includes a step of determining that an AF episode has occurred when each of biosignals of consecutive unit times of more than a preset minimum number is classified as atrial fibrillation and signal quality is determined to be good; a step of determining, after occurrence of an AF episode, that the AF episode has ended when each of biosignals of a preset minimum number of consecutive unit times is determined as non-atrial fibrillation; and a step of calculating a time from start of the AF episode to end of the AF episode as a duration time.

In one embodiment, the non-atrial fibrillation includes a case of a unit signal having good signal quality and normal sinus rhythm and a case of poor signal quality.

In one embodiment, when the analysis time is less than a preset minimum analysis time, the analysis time is treated as an overestimation error of an AF burden.

In accordance with another aspect of the present invention, provided is a device for analyzing atrial fibrillation including a biosignal collection unit for collecting a biosignal generated by a sensor; a preprocessing unit for removing noise from the collected biosignal; a signal quality estimation unit for estimating signal quality of the biosignal of a preset unit time; a signal analysis unit for classifying the biosignal of a preset unit time as atrial fibrillation (AF) or normal sinus rhythm (NSR); an episode determination unit for determining whether an AF episode has occurred and a duration time based on signal quality and AF classification results for biosignals of a plurality of unit times; and an AF burden estimation unit for calculating an atrial fibrillation burden based on whether the abnormal condition episode has occurred and the duration time.

In one embodiment, the AF burden estimation unit calculates an analysis time by adding duration times of analyzable unit signals among actually collected biosignals, calculates a sum of duration times of atrial fibrillation episodes occurring at the analysis time, and calculates a ratio of the sum of duration times of the atrial fibrillation episodes to the analysis time.

In one embodiment, the analyzable unit signals include a unit signal included in a minimum unit in which signal quality of each of biosignals of a preset minimum number of consecutive unit times is determined to be good; a unit signal included in a minimum unit in which signal quality of at least one of biosignals of a preset minimum number of consecutive unit times is determined to be good and the biosignal is determined as normal sinus rhythm; and all unit signals until it is determined that an AF episode has ended, after each of biosignals of consecutive unit times of more than a preset minimum number is classified as atrial fibrillation and signal quality is determined to be good, after an AF episode occurs, when each of biosignals of a preset minimum number of consecutive unit times is determined as non-atrial fibrillation.

In one embodiment, the episode determination unit determines that an AF episode has occurred when each of biosignals of consecutive unit times of more than a preset minimum number is classified as atrial fibrillation and signal quality is determined to be good; determines, after occurrence of an AF episode, that the AF episode has ended when each of biosignals of a preset minimum number of consecutive unit times is determined as non-atrial fibrillation; and calculates a time from start of the AF episode to end of the AF episode as a duration time.

In one embodiment, the non-atrial fibrillation includes a case of a unit signal having good signal quality and normal sinus rhythm and a case of poor signal quality.

In one embodiment, when the analysis time is less than a preset minimum analysis time, the AF burden estimation unit treats the analysis time as an overestimation error of an AF burden.

In one embodiment, the device for analyzing atrial fibrillation further include an output unit for outputting one or more of hourly/daily/weekly/monthly statistics of AF burdens, hourly/daily/weekly/monthly statistics of a total duration time of AF episodes, minimum/maximum duration times of atrial fibrillation episodes, hourly/daily/weekly/monthly statistics of a quartile of atrial fibrillation episodes, hourly/daily/weekly/monthly statistics of median/mean values of atrial fibrillation episodes, hourly/daily/weekly/monthly statistics of analysis times of AF burdens, and hourly/daily/weekly/monthly statistics of usage times (operating times).

In addition, although atrial fibrillation has been described as an example of the abnormal signal described in this specification, the present invention is not limited thereto, and the abnormal signal may include various abnormal physiological signals such as a high blood pressure signal and a hypoxia signal.

Advantageous Effects

According to the present invention, an abnormal condition burden can be accurately estimated by analyzing a biosignal obtained from a sensor interfaced with a subj ect.

Description of Drawings

FIG. 1 is a block diagram for explaining a device for analyzing atrial fibrillation according to an embodiment of the present invention.

FIG. 2 is a flowchart for explaining an analysis method using a device for analyzing atrial fibrillation according to an embodiment of the present invention.

FIG. 3 includes vertical lines for explaining a case defined as a minimum unit AF episode and a case not defined as a minimum unit AF episode according to an embodiment of the present invention.

FIG. 4 includes vertical lines for explaining the occurrence time of AF episodes over time according to an embodiment of the present invention.

FIG. 5 includes vertical lines for additionally explaining AF episodes over time according to an embodiment of the present invention.

FIG. 6 includes vertical lines for explaining the combination of unit signals capable of defining an atrial fibrillation episode according to an embodiment of the present invention.

FIG. 7 includes vertical lines for explaining a minimum analyzable time according to an embodiment of the present invention.

FIG. 8 is a block diagram illustrating the configuration of a device for analyzing atrial fibrillation according to another embodiment of the present invention.

Best Mode

Hereinafter, the present invention will be described in detail so that those skilled in the art can understand and easily practice the present invention through embodiments described with reference to the accompanying drawings.

In addition, in the following description of the present invention, a detailed description of known functions and configurations incorporated herein will be omitted when it may make the subject matter of the present invention unclear.

Prior to description of the present invention, in this specification, a unit signal refers to a signal collected for a preset unit time (e.g., 5 seconds, 10 seconds, 20 seconds, or the like).

An atrial fibrillation (AF) episode, which is an example of an abnormal signal among physiological abnormalities, means a clinically significant "event in which atrial fibrillation has occurred" based on a unit signal classified as atrial fibrillation (AF), as will be described later. That is, an atrial fibrillation episode is one event in which AF occurs and ends.

Here, physiological abnormalities are not limited to atrial fibrillation, and may include hypertension, hypoxia, and high fever.

In addition, an abnormal condition episode that lasts for a preset minimum time (e.g., 15 seconds, 20 seconds, 30 seconds, or the like) is called a minimum unit abnormal condition episode.

In this specification, "a minimum unit abnormal condition (e.g., AF) episode may be defined" means that it may be determined whether a corresponding minimum unit is an abnormal condition episode.

In this specification, "defined as a minimum unit abnormal condition episode" means that a corresponding minimum unit is an abnormal condition episode.

In this specification, "may not be defined as a minimum unit abnormal condition episode" means that a corresponding minimum unit is not an abnormal condition episode.

In this specification, "a minimum unit abnormal condition episode may not be defined" means that it may not be determined whether a corresponding minimum unit is an abnormal condition episode.

[Description of Symbols]

| 100: | DEVICE FOR ANALYZING ABNORMAL CONDITIONS |
| 110: | BIOSIGNAL COLLECTION UNIT |
| 120: | PREPROCESSING UNIT |
| 130: | SIGNAL QUALITY ESTIMATION UNIT |
| 140: | SIGNAL ANALYSIS UNIT |
| 150: | UNIT SIGNAL DETERMINATION UNIT |
| 160: | EPISODE DETERMINATION UNIT |
| 170: | BURDEN ESTIMATION UNIT |

[Mode for Invention]

Hereinafter, embodiments will be described in detail with reference to the accompanying drawings.

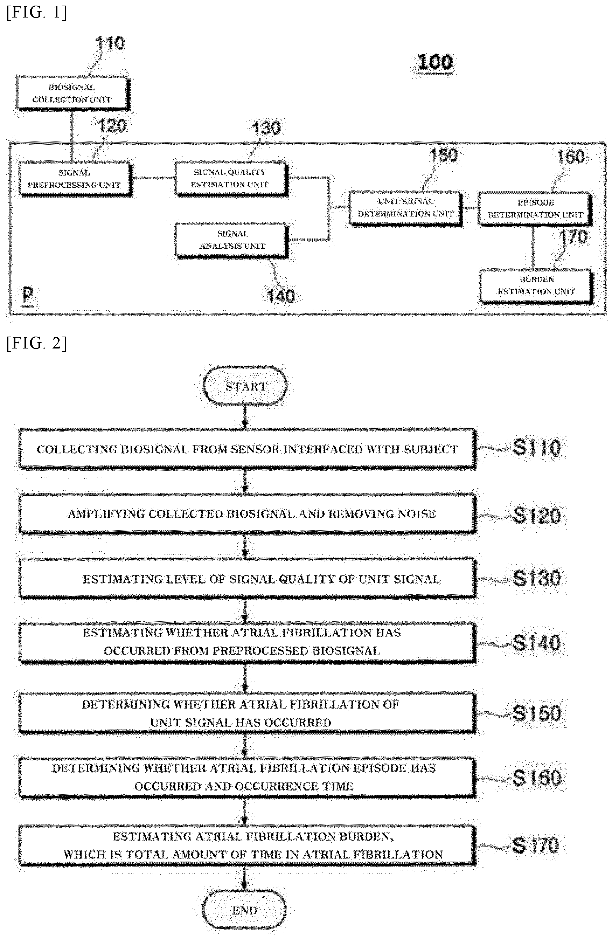

FIG. 1 is a block diagram for explaining a device for analyzing atrial fibrillation according to an embodiment of the present invention, and FIG. 2 is a flowchart for explaining an analysis method using a device for analyzing atrial fibrillation according to an embodiment of the present invention.

As shown in FIG. 1, a device for analyzing atrial fibrillation 100 includes a biosignal collection unit 110 and a processor P1. The processor P1 may include a preprocessing unit 120, a signal quality estimation unit 130, a signal analysis unit 140, a unit signal determination unit 150, an episode determination unit 160, and a burden estimation unit 170.

Steps S100 to S170 included in the atrial fibrillation analysis method of FIG. 2 may be performed using the atrial fibrillation analysis device 100 of FIG. 1.

In step S110, the biosignal collection unit 110 may include a sensor for measuring a biosignal, such as photoplethysmography (PPG), and obtain a biosignal such as a pulse wave signal from the sensor interfaced with a subject. The sensor may include a light source for radiating light to a subject and a detector for detecting light radiated by the light source and scattered or reflected from the surface of the subject or biological tissues such as blood vessels. For example, the PPG sensor may be a ring-type sensor that continuously collects and stores biosignals from blood vessels located in the finger.

The light source may include one or more light-emitting diodes (LEDs), laser diodes (LDs), or phosphors. In this case, when one or more light sources are configured, each light source may emit light of a different wavelength.

The detector may include one or more photo diodes, photo transistors (PTrs), or image sensors (e.g., CMOS image sensors). When one or more detectors are configured, the light sources may be placed at different distances from each detector. In addition, the pulse wave signal may mean a PPG signal, without being limited thereto.

In addition, in another embodiment, the biosignal collection unit 110 may receive a user's pulse wave signal from an external device by communicating with the external device. For example, the biosignal collection unit 110 may receive a biosignal such as user's pulse waves from the external device through Bluetooth communication, Bluetooth Low Energy (BLE) communication, Near Field Communication (NFC), WLAN communication, Zigbee communication, Infrared Data Association (IrDA) communication, Wi-Fi Direct (WFD) communication, ultra-wideband (UWB) communication, Ant+ communication, WIFI communication, or Radio Frequency Identification (RFID) communication.

In step S120, the signal preprocessing unit 120 may include a filter, and the collected biosignal is amplified and noise is eliminated. Since the biosignal collected through the sensor may contain elements that make measurement inaccurate, such as noise, the signal preprocessing unit 120 removes elements that make measurement inaccurate.

In one embodiment, the signal preprocessing unit 120 may remove noise by exceptionally processing signals exceeding a predetermined threshold based on the size of a unit signal.

In one embodiment, the signal preprocessing unit 120 may extract a waveform of a signal and extract features from the extracted waveform.

In step S130, the signal quality estimation unit 130 estimates the level of signal quality (hereinafter referred to as SQ) of the preprocessed unit signal.

In one embodiment, the signal quality estimation unit 130 operates based on a machine learning-based algorithm for quality estimation. For example, signal quality may be estimated based on the characteristics of a biosignal from which noise has been removed. As the similarity between the characteristics of a biosignal and a reference signal provided based on a machine learning-based algorithm generated based on correlation with signal quality to be estimated increases, signal quality may be estimated to be good. The range of signal quality levels is between 0 and 1. As the signal quality level approaches 1, the signal quality increases. As the machine learning-based algorithm, a pre-generated machine learning algorithm received from an external device through wired/wireless communication may be used.

The signal quality estimation unit 130 determines that the signal quality is good (SQ Good) when the estimated signal quality level is equal to or greater than a preset threshold, or determines that the signal quality is poor (SQ Poor) when the estimated signal quality level is less than a preset threshold.

In step S140, the signal analysis unit 140 estimates whether an abnormal signal, such as atrial fibrillation (AF), has occurred from the preprocessed biosignal. That is, the signal analysis unit 140 determines whether the preprocessed biosignal corresponds to atrial fibrillation, which is an abnormal signal, or normal sinus rhythm (NSR), which is a normal signal.

In one embodiment, the signal analysis unit 140 operates based on a machine learning algorithm for classifying abnormal signals (e.g., AF).

In step S150, the unit signal determination unit 150 determines whether an abnormal condition has occurred with respect to a unit signal based on the results of the signal quality estimation unit 130 and the signal analysis unit 140. For example, the unit signal determination unit 150 determines the reliability of the signal classification result of the signal analysis unit 140 based on the result of the signal quality estimation unit 130. That is, only when signal quality is good as a result of the signal quality estimation unit 130, the analysis result of the signal analysis unit 140 is trusted. As a result of the signal quality estimation unit 130, when signal quality is poor, the reliability of the signal itself is low, so the result of the signal analysis unit 140, which analyzes the signal with low reliability, is also not reliable, so the corresponding unit signal is determined as "unknown".

In summary, the unit signal determination unit 150 determines a biosignal analysis result as "unknown" due to low reliability when signal quality is low (poor signal). When signal quality is high (good signal), the unit signal determination unit 150 finally determines whether a biosignal is an abnormal signal such as atrial fibrillation (AF) or a normal signal such as normal sinus rhythm (NSR) according to the abnormal signal classification result of the signal analysis unit 140.

In step S160, an episode analysis unit 160 determines occurrence and duration of an abnormal condition episode during a certain period based on unit signals analyzed by the unit signal determination unit 150.

As an embodiment, an episode in which unit times determined as atrial fibrillation by the unit signal determination unit 150 last for a preset minimum time (e.g., 15 seconds, 20 seconds, 30 seconds, or the like) is defined as a minimum unit AF episode.

In step S170, the burden estimation unit 170 estimates an abnormal condition burden, such as an atrial fibrillation (AF) burden.

The atrial fibrillation (AF) burden is calculated by Equation 1 below.

[Equation 1]

$$\text{AF burden (\%)} = \frac{\text{Total AF episode duration time}}{\text{Analysis time}}$$

Here, the analysis time is the total duration time of analyzable unit signals among actually collected biosignals. Here, "analyzable" means that it may be determined whether or not a corresponding unit signal is included in an AF episode. In other words, the analysis time means the total time of unit signals capable of determining whether or not the unit signals are included in an AF episode. When it may be determined whether a specific unit signal is included in an AF episode, the corresponding unit signal is classified as "analyzable". When it may not be determined whether a specific unit signal is included in an AF episode, the corresponding unit signal is classified as "non-Analyzable". For example, when signals are collected for 5 minutes and all unit signals collected are assumed to be "Poor signal (SQ Poor)", since it is not known whether AF has occurred for all collected unit signals, it is also impossible to determine whether each unit signal includes an AF episode. Accordingly, the analysis time for the collected 5 minutes is zero. That is, the analysis time is the total duration time of each unit signal that may determine "whether a unit signal is included in an AF episode".

That is, the burden estimation unit 170 determines, as analyzable biosignals, a unit signal included in a minimum unit in which signal quality of each of biosignals of a preset minimum number of consecutive unit times is determined to be good; a unit signal included in a minimum unit in which signal quality of at least one of biosignals of a preset minimum number of consecutive unit times is determined to be good and the biosignal is determined as a normal signal such as normal sinus rhythm; and all unit signals until it is determined that an AF episode has ended, after each of biosignals of consecutive unit times of more than a preset minimum number is classified as an abnormal signal such as atrial fibrillation and signal quality is determined to be good, after an abnormal condition episode, e.g., AF episode, occurs, when each of biosignals of a preset minimum number of consecutive unit times is determined as non-atrial fibrillation.

As described above, an AF episode has a minimum unit. Accordingly, "an AF episode may be defined" eventually leads to "a minimum unit AF episode, which is the minimum unit of an AF episode, may be defined".

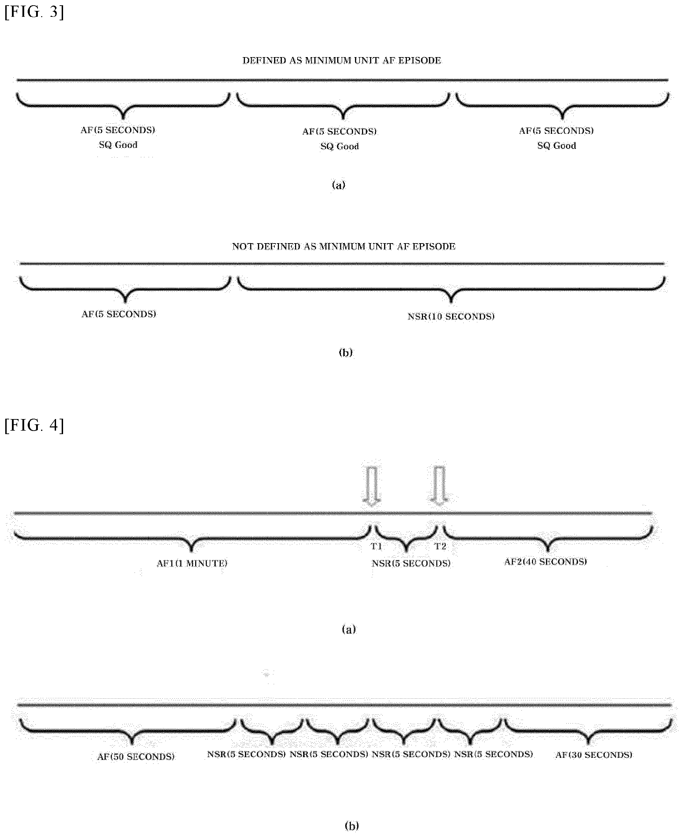

Hereinafter, some steps of the atrial fibrillation analysis method of FIG. 2 will be described in detail with reference to FIGS. 3 to 7. For convenience of description, atrial fibrillation analysis is described as an example, but the present invention is not limited thereto. Atrial fibrillation described in the following embodiments is an abnormal signal in another modified embodiment, and normal sinus rhythm is a normal signal in another modified embodiment.

FIG. 3 includes vertical lines for explaining a case defined as a minimum unit AF episode and a case not defined as a minimum unit AF episode according to an embodiment of the present invention.

FIG. 3A includes vertical lines for explaining a case defined as a minimum unit AF episode, and FIG. 3B includes vertical lines for explaining a case not defined as a minimum unit AF episode.

For example, assuming that the duration time of a unit signal is 5 seconds and the duration time of a minimum unit AF episode is 15 seconds, the duration time of the minimum unit AF episode is satisfied when the unit signal is three or more times. In addition, assuming that the duration time of a unit signal is 20 seconds and the duration time of a minimum unit AF episode is 100 seconds, the duration time of the minimum unit AF episode is satisfied when the unit signal is five or more times. In this way, the consecutive number of unit signals satisfying the duration time of the minimum unit AF episode is referred to as the minimum number of times.

As shown in FIG. 3A, a unit signal determined as atrial fibrillation continues three times in a row, and the episode analysis unit 160 determines an event that satisfies the minimum number of times or the minimum time as an AF episode.

As shown in FIG. 3B, when a unit signal determined as atrial fibrillation does not satisfy the minimum number of times or the minimum time, e.g., when a unit signal determined as NSR of 10 seconds lasts 2 times, i.e., 10 seconds, after lasting 1 time, i.e., 5 seconds, the corresponding event is not determined as an AF episode.

As such, the episode analysis unit (160 in FIG. 1) ignores a noncontiguous time and calculates an AF episode occurrence time when the noncontiguous time is less than a preset minimum time even when the AF episode is sensed in a non-continuous manner.

FIG. 4 includes vertical lines for explaining the occurrence time of AF episodes over time according to an embodiment of the present invention.

As shown in FIG. 4A, a case in which NSR occurs at a point T1 where an atrial fibrillation AF signal (AF1) lasts for 1 minute and an atrial fibrillation AF signal (AF2) lasts for 40 seconds at a point T2 where NSR lasts for 5 seconds is considered.

When the time between discontinuous points T1 and T2 of an AF signal (AF1) and an AF signal (AF2) is less than a preset minimum time (e.g., 20 seconds) for defining an episode, the episode analysis unit 160 ignores discontinuous time. In FIG. 4A, discontinuous time is ignored because the time between T1 and T2 is 5 seconds, which is less than a preset minimum time of 20 seconds. Accordingly, an AF episode occurrence time is 1 minute 45 seconds, which is the sum of an AF signal (AF1) duration time, an NSR duration time, and an AF signal (AF2) duration time.

Here, the reason why the episode analysis unit 160 ignores an NSR time less than the minimum time is to correct errors in sensing and analysis.

Specifically, when the signal analysis unit 140 determines that an arbitrary biosignal is NSR, the signal analysis unit 140 may correctly determine actual NSR as NSR and may misjudge actual AF as NSR.

However, based on clinical information, after an AF episode has occurred, after a normal rhythm (NSR occurrence) is restored in a very short time, the probability of an AF episode occurring again is extremely low, so a biosignal determined as NSR for a very short time between AF episodes is ignored. Here, the very short time may be less than about 20 seconds as a meaningful time for clinical judgment. Accordingly, in FIG. 4A, the biosignal judged as NSR for a very short time of 5 seconds between AF1 and AF2 is determined to be an erroneous estimation of the actual AF generation signal as NSR, and the biosignal is ignored. In addition, in the present specification, a meaningful time or a very short time is not limited to 20 seconds or 5 seconds.

Here, when the probability that the signal analysis unit 140 correctly judges the actual NSR as NSR is P1, and the probability of misjudging the actual AF as NSR is P2, the signal analysis unit 140 may perform judgement by comparing a value obtained by multiplying the probability of an actual AF signal and the probability (P2) of misjudging an actual AF signal as an NSR signal and a value obtained by multiplying the probability that a signal is an actual NSR signal and the probability (P1) of correctly determining an actual NSR as NSR. Here, the probability that a signal is an actual AF signal and the probability that a signal is an actual NSR signal may be set in advance according to clinical statistics.

As another example, as shown in FIG. 4B, when an atrial fibrillation AF signal (AF1) lasts for 50 seconds, and an atrial fibrillation AF signal (AF2) lasts for 30 seconds after NSR lasts for 4 consecutive times for 5 seconds, since the time of NSR is more than 20 seconds, which is a preset minimum time, the episode analysis unit 160 analyzes the case of FIG. 4B into a total of two episodes: a first episode with an occurrence time of 50 seconds and a second episode with an occurrence time of 30 seconds that occurred 20 seconds later. In addition, the preset minimum time is not limited to 20 seconds, and may be set to other times such as 10 seconds or 30 seconds.

As described above, when the unit time NSR signal occurs 4 times in a row, the probability that 4 consecutive NSR signals were real AF is a value obtained by calculating an equation of (Probability of real AF signal × Probability (P2) of misjudging real AF signal as NSR signal)[4]. Since (P2)[4] is a value close to 0, the value obtained by calculating the equation of (Probability of real AF signal × Probability (P2) of misjudging real AF signal as NSR signal)[4] is also a value close to 0. In other words, it is interpreted that the probability that the actual AF signal is consecutively misjudged as NSR is very low. Accordingly, the probability of actual NSR occurring in at least one of four NSR signals is higher than the probability of incorrectly determining four signals as NSR in succession. Accordingly, when a predetermined number, e.g., 4 or more, of consecutive NSR signals occur, it may be determined that reliable NSR exist.

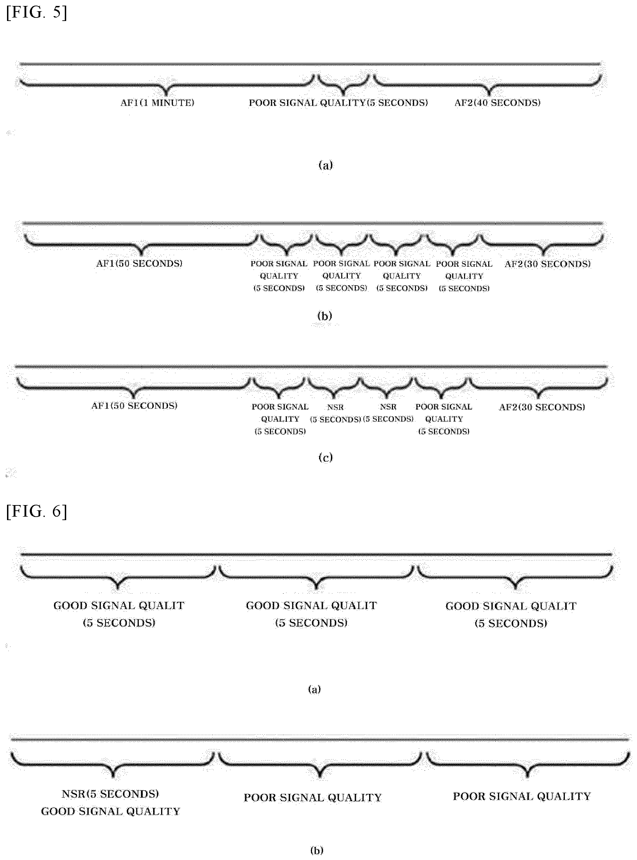

FIG. 5 includes vertical lines for additional explanation of AF episodes over time according to an embodiment of the present invention. Hereinafter, AF episodes described with reference to FIG. 5 is an embodiment, and the present invention is not limited thereto, and the AF episodes may include various abnormal physiological condition episodes.

FIGS. 3 and 4 describe a case of good signal quality, but FIG. 5 describes a case in which poor signal quality is included.

As shown in FIG. 5A, a case in which an atrial fibrillation AF signal lasts for 1 minute, a unit signal (poor signal quality) determined to have poor signal quality lasts for 5 seconds, and then an atrial fibrillation AF signal lasts for 40 seconds is described.

Since poor signal quality between the AF signal (AF1) and the AF signal (AF2) occurred for 5 seconds, which is a very short time less than a minimum time of 20 seconds, the episode analysis unit 160 has no difficulty in determining that the AF signal is a signal generated within 5 seconds of poor signal quality. That is, a signal generated in a very short time (e.g., 5 seconds) with poor signal quality is determined according to the result of classification of the front and rear signals.

In the case of FIG 5A, the AF episode occurrence time is 1 minute 45 seconds, which is the sum of AF signal (AF1) duration time, poor signal quality duration time, and AF signal (AF2) duration time. More specifically, since the probability of an AF episode occurring again after an AF episode occurs and NSR occurs for a very short time is extremely low, even when it is impossible to determine whether it is AF due to signal failure between AF episodes, the very short time may be regarded as AF.

As another example, as shown in FIG. 5B, a case in which the atrial fibrillation AF signal (AF1) lasts for 50 seconds, the signal of poor signal quality continues 4 consecutive times for 5 seconds, and the atrial fibrillation AF signal (AF2) lasts for 30 seconds is described.

Since the time of poor signal quality is more than 20 seconds, which is a preset minimum time, the episode analysis unit 160 meaningfully interprets the 20-second period of poor signal quality, and analyzes the case of FIG 5B into two episodes: the first episode with an occurrence time of 50 seconds and the second episode with an occurrence time of 30 seconds that occurs 20 seconds later.

As another example, as shown in FIG. 5C, a case in which an atrial fibrillation AF signal (AF1) is generated and lasts for 50 seconds, a signal with poor signal quality lasts for 5 seconds, a normal sinus rhythm (NSR) with good signal quality lasts for 10 seconds, a signal with poor signal quality lasts for 5 seconds, and an atrial fibrillation AF signal (AF2) lasts for 30 seconds is described.

Signals with poor signal quality and normal sinus rhythm signals are called non-atrial fibrillation signals, and the non-atrial fibrillation signals are processed similarly to the signals with poor signal quality. Accordingly, since the time when the non-atrial fibrillation signal continuously occurs is 20 seconds or more, which is a preset minimum time, the episode analysis unit 160 meaningfully interprets the 20-second time period in which the non-atrial fibrillation signals occur consecutively, and analyzes the case of FIG. 5C into two episodes: the first episode with an occurrence time of 50 seconds and the second episode with an occurrence time of 30 seconds that occurs 20 seconds later.

To summarize, in the episode analysis unit 160, after occurrence of the minimum unit atrial fibrillation episode, at least consecutive minimum number of times (4 times), each unit signal is "signal quality good (SQ Good) & NSR" or "poor signal quality (SQ Poor)", the atrial fibrillation episode ends.

FIG. 6 includes vertical lines for explaining the combination of unit signals capable of defining an atrial fibrillation episode according to an embodiment of the present invention.

FIGS. 6A and 6B illustrate combinations of various unit signals included in the minimum unit of an AF episode having a duration time of 15 seconds.

As shown in FIG. 6A, when all unit signals included in a minimum unit have good signal quality, it is analyzed as "a minimum unit AF episode may be defined". Here, "a minimum unit AF episode may be defined" means "it may be determined whether a corresponding minimum unit is an AF episode". Conversely, "a minimum unit AF episode may not be defined" means "it may not be determined whether a corresponding minimum unit is an AF episode".

As shown in FIG. 6B, when at least one of unit signals included in the minimum unit is "good signal quality & normal sinus rhythm (NSR)", regardless of the signal quality level of the remaining unit signals or atrial fibrillation, it is analyzed as "the corresponding minimum unit is not an AF episode".

In summary,

1. "A minimum unit AF episode may be defined" means "it may be determined whether a corresponding minimum unit is an AF episode".

2. "Defined as a minimum unit AF episode" means "a corresponding minimum unit is an AF episode".

3. "May not be defined as a minimum unit AF episode" means "a corresponding minimum unit is not an AF episode".

4. "A minimum unit AF episode may not be defined" means "it may not be determined whether a corresponding minimum unit is an AF episode".

For example, 1) when the signal quality of all unit signals included in a minimum unit is good and all unit signals are determined to be AF, a corresponding minimum unit is determined as an AF episode. 2) When at least one of unit signals included in a minimum unit is "good signal quality & normal sinus rhythm", it is determined that a corresponding minimum unit is not an AF episode.

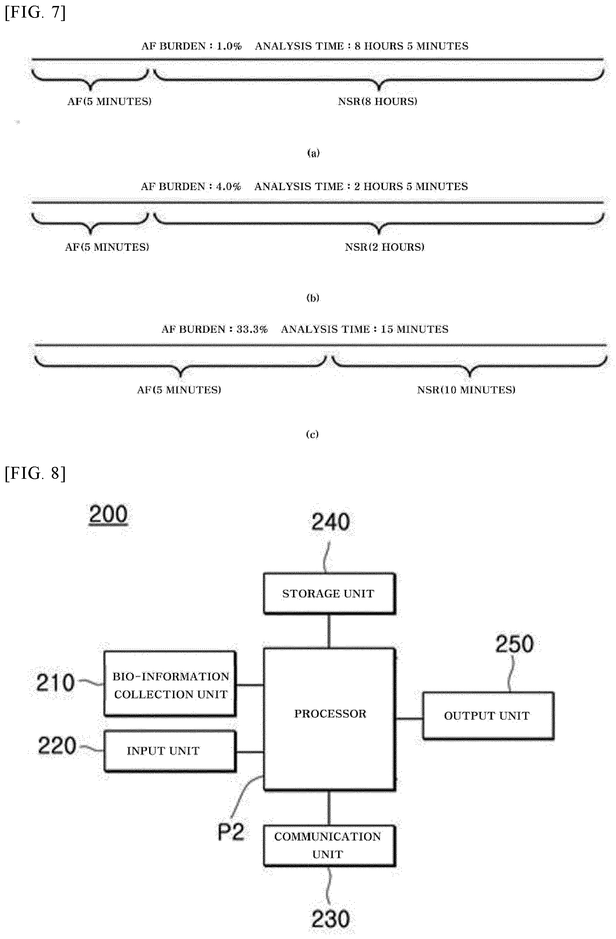

FIG. 7 includes vertical lines for explaining a minimum analyzable time according to an embodiment of the present invention.

As described above, the atrial fibrillation burden is a numerical value expressed as a relative ratio of the total time of an atrial fibrillation episode to the analysis time. Accordingly, the atrial fibrillation burden is affected by the analysis time in addition to the total duration time of the atrial fibrillation burden. In particular, when the analysis time is very short, the atrial fibrillation burden may be distorted.

FIGS. 7A, 7B, and 7C show examples in which the atrial fibrillation episode duration time is the same as 5 minutes, but the analysis time is different.

In FIG. 7A, the atrial fibrillation episode duration time is 5 minutes and the analysis time is 8 hours and 5 minutes, so the atrial fibrillation burden is 1.0 %.

In FIG. 7B, the atrial fibrillation episode duration time is 5 minutes and the analysis time is 2 hours and 5 minutes, so the atrial fibrillation burden is 4.0 %.

In FIG. 7A, the atrial fibrillation episode duration time is 5 minutes and the analysis time is 15 minutes, so the atrial fibrillation burden is 33.3 %.

As can be seen by comparing FIGS. 7A, 7B, and 7C, the atrial fibrillation burden differs according to the analysis time even when the atrial fibrillation episode duration time is the same. In particular, in the case of FIG. 7C, it can be confirmed that the atrial fibrillation burden is overestimated at 33.3% because the analysis time is 15 minutes, which is very short to measure a clinically significant burden.

To solve the problem of overestimation, the AF burden estimation unit 170 defines minimum analysis time that satisfies the following two conditions to be clinically meaningful.

First, the AF burden value of a minimum unit AF episode compared to a minimum analysis time is less than 1 %.

Second, an analysis time of more than a preset time that may be sufficiently collected during a day is, for example, 3 hours (Here, the analysis time longer than the preset time may be adjusted according to the purpose of atrial fibrillation burden analysis and sensor sensitivity).

That is, the AF burden estimation unit 170 calculates an atrial fibrillation burden when the conditions of the two minimum analysis times are satisfied. The AF burden estimation unit 170 may process the overestimation error of a burden when the minimum analysis time is not satisfied.

FIG. 8 is a block diagram illustrating the configuration of a device for analyzing atrial fibrillation according to another embodiment of the present invention.

Referring to FIG. 8, a device 200 for analyzing atrial fibrillation may include a biosignal collection unit 210, a processor P2, an input unit 220, a storage unit 230, a communication unit 240, and an output unit 250. Here, since the biosignal collection unit 210 and the processor P2 perform the same functions as the biosignal collection unit 110 and the processor P1 described above with reference to FIG. 1, description will be given focusing on non-overlapping configurations.

The input unit 220 converts user's input operation into an input signal and transmits the input signal to the processor P2. For example, the input unit 220 will be implemented as a keyboard, a dome switch, a jog wheel, a touch sensor on a touch screen, a touch pad, a keypad, voice input, input processing devices used in the present and in the past, and input processing devices to be used in the future. For example, the input unit 220 may receive an atrial fibrillation burden information provision request input and transmit the input to the processor P2.

The storage unit 230 may store information received by the communication unit 240 and information and program codes necessary for operation of the device 200 for analyzing atrial fibrillation.

The communication unit 240 exchanges data with an external device. For example, the communication unit 240 may transmit data analyzed by a processor P to an external device. Communication technology used by the communication unit 240 may vary depending on the type of communication network or other circumstances.

The output unit 250 may be implemented as liquid crystal display (LCD), light-emitting diode (LED), organic light-emitting diode (OLED), a projector, display devices used in the present and in the past, and display devices to be used in the future. For example, the output unit 250 may output information about a biosignal, whether an atrial fibrillation episode has occurred, a duration time, an alarm according to occurrence of atrial fibrillation, or an atrial fibrillation burden.

In addition, the output unit 250 may output a result in response to the atrial fibrillation burden information provision request input of an input unit 210 according to the instruction of the processor P2. In addition, the output unit 250 may display an interface page for providing information or a result page for providing information. In another embodiment, information may be transmitted to a user through voice output or vibration instead of screen output.

In one embodiment, the processor P2 may calculate an atrial fibrillation burden in response to an atrial fibrillation burden information provision request input, and output the calculated result through the output unit 250. At this time, when an analysis time of the burden of the atrial fibrillation episode is less than the total minimum analysis time, the processor P2 may output a warning message together with an AF burden.

For example, the warning message may be "There is a possibility that the atrial fibrillation burden value was measured higher than the actual value because the analysis time was not sufficient. Please increase usage time to collect sufficient analysis time".

The processor P2 may calculate burden-related statistics in addition to the atrial fibrillation burden, and output the calculated statistics through the output unit 250.

For example, the processor P2 may analyze and provide the hourly/daily/weekly/monthly statistics of an atrial fibrillation burden, the hourly/daily/weekly/monthly statistics of a total duration time of an AF episode, the minimum/maximum duration time of an atrial fibrillation episode, the hourly/daily/weekly/monthly statistics of a quartile of an atrial fibrillation episode, the hourly/daily/weekly/monthly statistics of the median/mean values of atrial fibrillation episodes, the hourly/daily/weekly/monthly statistics of analysis time of an atrial fibrillation episode, and the hourly/daily/weekly/monthly statistics of usage time (operating time).

The apparatus described above may be implemented as a hardware component, a software component, and/or a combination of hardware components and software components. For example, the apparatus and components described in the embodiments may be achieved using one or more general purpose or special purpose computers, such as, for example, a processor, a controller, an arithmetic logic unit (ALU), a digital signal processor, a microcomputer, a field programmable gate array (FPGA), a programmable logic unit (PLU), a microprocessor, or any other device capable of executing and responding to instructions. The processing device may execute an operating system (OS) and one or more software applications executing on the operating system. In addition, the processing device may access, store, manipulate, process, and generate data in response to execution of the software. For ease of understanding, the processing apparatus may be described as being used singly, but those skilled in the art will recognize that the processing apparatus may include a plurality of processing elements and/or a plurality of types of processing elements. For example, the processing apparatus may include a plurality of processors or one processor and one controller. Other processing configurations, such as a parallel processor, are also possible.

The software may include computer programs, code, instructions, or a combination of one or more of the foregoing, configure the processing apparatus to operate as desired, or command the processing apparatus, either independently or collectively. In order to be interpreted by a processing device or to provide instructions or data to a processing device, the software and/or data may be embodied permanently or temporarily in any type of a machine, a component, a physical device, a virtual device, a computer storage medium or device, or a transmission signal wave. The software may be distributed over a networked computer system and stored or executed in a distributed manner. The software and data may be stored in one or more computer-readable recording media.

The methods according to the embodiments of the present invention may be implemented in the form of a program command that can be executed through various computer means and recorded in a computer-readable medium. The computer-readable medium can store program commands, data files, data structures or combinations thereof. The program commands recorded in the medium may be specially designed and configured for the present invention or be known to those skilled in the field of computer software. Examples of a computer-readable recording medium include magnetic media such as hard disks, floppy disks and magnetic tapes, optical media such as CD-ROMs and DVDs, magneto-optical media such as floptical disks, or hardware devices such as ROMs, RAMs and flash memories, which are specially configured to store and execute program commands. Examples of the program commands include machine language code created by a compiler and high-level language code executable by a computer using an interpreter and the like.

Although the present invention has been described with reference to limited embodiments and drawings, it should be understood by those skilled in the art that various changes and modifications may be made therein. For example, the described techniques may be performed in a different order than the described methods, and/or components of the described systems, structures, devices, circuits, etc., may be combined in a manner that is different from the described method, or appropriate results may be achieved even when replaced by other components or equivalents.

Therefore, other embodiments, other examples, and equivalents to the claims are within the scope of the following claims.

[Industrial Applicability]

The present invention can provide a method of accurately estimating an abnormal condition, such as an atrial fibrillation burden, by analyzing a biosignal obtained from a sensor interfaced with a subject and a device therefor.

**Claims**

1. A method of analyzing abnormal physiological signals, wherein the method is performed using a device for analyzing abnormal physiological signals and comprises a step of removing noise from a biosignal generated by a sensor; a step of estimating signal quality of the biosignal of a preset unit time; a step of classifying the biosignal of a preset unit time as an abnormal signal or a normal signal; a step of determining whether an abnormal condition episode has occurred and a duration time based on signal quality and abnormal signal classification results for biosignals of a plurality of unit times; and a step of calculating an abnormal condition burden based on whether the abnormal condition episode has occurred and the duration time.

2. The method according to claim 1, wherein the step of calculating an abnormal condition burden comprises a step of calculating an analysis time by adding duration times of analyzable unit signals among actually collected biosignals; a step of calculating a sum of duration times of abnormal condition episodes occurring at the analysis time; and a step of calculating a ratio of the sum of duration times of the abnormal condition episodes to the analysis time.

3. The method according to claim 2, wherein the analyzable unit signals comprise a unit signal comprised in a minimum unit in which signal quality of each of biosignals of a preset minimum number of consecutive unit times is determined to be good; a unit signal comprised in a minimum unit in which signal quality of at least one of biosignals of a preset minimum number of consecutive unit times is determined to be good and the biosignal is determined as a normal signal; and all unit signals until it is determined that an abnormal condition episode has ended, after each of biosignals of consecutive unit times of more than a preset minimum number is classified as an abnormal signal and signal quality is determined to be good, after an abnormal condition episode occurs, when each of biosignals of a preset minimum number of consecutive unit times is determined as a non-abnormal signal.

4. The method according to claim 1, wherein the step of determining whether an abnormal condition episode has occurred and a duration time comprises a step of determining that an abnormal condition episode has occurred when each of biosignals of consecutive unit times of more than a preset minimum number is classified as an abnormal signal and signal quality is determined to be good; a step of determining, after occurrence of an abnormal condition episode, that the abnormal condition episode has ended when each of biosignals of a preset minimum number of consecutive unit times is determined as a non-abnormal signal; and a step of calculating a time from start of the abnormal condition episode to end of the abnormal condition episode as a duration time.

5. The method according to claim 3 or 4, wherein the non-abnormal signal comprises a signal comprising a unit signal having good signal quality and a normal signal; and a signal having poor signal quality.

6. The method according to claim 2, wherein, in the step of calculating an analysis time, when the analysis time is less than a preset minimum analysis time, the analysis time is treated as an overestimation error of an abnormal condition burden.

7. The method according to claim 1, wherein the abnormal signal is atrial fibrillation (AF), the normal signal is normal sinus rhythm (NSR), and the abnormal condition episode is an AF episode.

8. A device for analyzing abnormal physiological signals, comprising:

   a biosignal collection unit for collecting a biosignal generated by a sensor;
   a preprocessing unit for removing noise from the collected biosignal;
   a signal quality estimation unit for estimating signal quality of the biosignal of a preset unit time;
   a signal analysis unit for classifying the biosignal of a preset unit time as an abnormal signal or a normal signal;
   an episode determination unit for determining whether an abnormal condition episode has occurred and a duration time based on signal quality and abnormal signal classification results for biosignals of a plurality of unit times; and
   an abnormal signal burden estimation unit for calculating an abnormal condition burden based on whether the abnormal condition episode has occurred and the duration time.

9. The device according to claim 8, wherein the abnormal signal burden estimation unit calculates an analysis time by adding duration times of analyzable unit signals among actually collected biosignals, calculates duration times of abnormal condition episodes occurring at the analysis time, and calculates a ratio of a sum of duration times of the abnormal condition episodes to the analysis time.

10. The device according to claim 9, wherein the abnormal condition burden estimation unit determines, as analyzable unit signals, a unit signal comprised in a minimum unit in which signal quality of each of biosignals of a preset minimum number of consecutive unit times is determined to be good; a unit signal comprised in a minimum unit in which signal quality of at least one of biosignals of a preset minimum number of consecutive unit times is determined to be good and the biosignal is determined as a normal signal; and all unit signals until it is determined that an abnormal condition episode has ended, after each of biosignals of consecutive unit times of more than a preset minimum number is classified as an abnormal signal and signal quality is determined to be good, after an abnormal condition episode occurs, when each of biosignals of a preset minimum number of consecutive unit times is determined as a non-abnormal signal.

11. The device according to claim 8, wherein the episode determination unit determines that an abnormal condition episode has occurred when each of biosignals of consecutive unit times of more than a preset minimum number is classified as an abnormal signal and signal quality is determined to be good; determines, after occurrence of an abnormal condition episode, that the abnormal condition episode has ended when each of biosignals of a preset minimum number of consecutive unit times is determined as a non-abnormal signal; and calculates a time from start of the abnormal condition episode to end of the abnormal condition episode as a duration time.

12. The device according to claim 10 or 11, wherein the non-abnormal signal comprises a case of a unit signal having good signal quality and a normal signal and a case of poor signal quality.

13. The device according to claim 9, wherein, when the analysis time is less than a preset minimum analysis time, the abnormal signal burden estimation unit treats the analysis time as an overestimation error of an abnormal signal burden.

14. The device according to claim 9, further comprising an output unit for outputting one or more of hourly/daily/weekly/monthly statistics of abnormal condition burdens, hourly/daily/weekly/monthly statistics of a total duration time of abnormal condition episodes, minimum/maximum duration times of abnormal condition episodes, hourly/daily/weekly/monthly statistics of a quartile of abnormal condition episodes, hourly/daily/weekly/monthly statistics of median/mean values of abnormal condition episodes, hourly/daily/weekly/monthly statistics of analysis times of abnormal condition episodes, and hourly/daily/weekly/monthly statistics of usage times (operating times).

[FIG. 1]

[FIG. 2]

[FIG. 3]

DEFINED AS MINIMUM UNIT AF EPISODE

AF (5 SECONDS)
SQ Good

AF (5 SECONDS)
SQ Good

AF (5 SECONDS)
SQ Good

(a)

NOT DEFINED AS MINIMUM UNIT AF EPISODE

AF (5 SECONDS)

NSR (10 SECONDS)

(b)

[FIG. 4]

AF1 (1 MINUTE)

T1

NSR (5 SECONDS)

T2

AF2 (40 SECONDS)

(a)

AF (50 SECONDS)

NSR (5 SECONDS) NSR (5 SECONDS) NSR (5 SECONDS) NSR (5 SECONDS)

AF (30 SECONDS)

(b)

[FIG. 5]

AF1(1 MINUTE)   POOR SIGNAL QUALITY(5 SECONDS)   AF2(40 SECONDS)

(a)

AF1(50 SECONDS)   POOR SIGNAL QUALITY (5 SECONDS)   POOR SIGNAL QUALITY (5 SECONDS)   POOR SIGNAL QUALITY (5 SECONDS)   POOR SIGNAL QUALITY (5 SECONDS)   AF2(30 SECONDS)

(b)

AF1(50 SECONDS)   POOR SIGNAL QUALITY (5 SECONDS)   NSR (5 SECONDS)   NSR (5 SECONDS)   POOR SIGNAL QUALITY (5 SECONDS)   AF2(30 SECONDS)

(c)

[FIG. 6]

GOOD SIGNAL QUALIT (5 SECONDS)   GOOD SIGNAL QUALIT (5 SECONDS)   GOOD SIGNAL QUALIT (5 SECONDS)

(a)

NSR(5 SECONDS) GOOD SIGNAL QUALITY   POOR SIGNAL QUALITY   POOR SIGNAL QUALITY

(b)

[FIG. 7]

AF BURDEN : 1.0%    ANALYSIS TIME : 8 HOURS 5 MINUTES

AF(5 MINUTES)                                    NSR(8 HOURS)

(a)

AF BURDEN : 4.0%    ANALYSIS TIME : 2 HOURS 5 MINUTES

AF(5 MINUTES)                                    NSR(2 HOURS)

(b)

AF BURDEN : 33.3%    ANALYSIS TIME : 15 MINUTES

AF(5 MINUTES)                                    NSR(10 MINUTES)

(c)

[FIG. 8]

<u>200</u>                                    240

STORAGE UNIT

210  BIO-INFORMATION
     COLLECTION UNIT

                                    250

                PROCESSOR        OUTPUT UNIT

220  INPUT UNIT

        P2

            COMMUNICATION
            UNIT

                230

## INTERNATIONAL SEARCH REPORT

| | International application No. |
|---|---|
| | **PCT/KR2021/013536** |

### A. CLASSIFICATION OF SUBJECT MATTER

**A61B 5/361**(2021.01)i; **A61B 5/363**(2021.01)i; **A61B 5/00**(2006.01)i

According to International Patent Classification (IPC) or to both national classification and IPC

### B. FIELDS SEARCHED

Minimum documentation searched (classification system followed by classification symbols)

A61B 5/361(2021.01); A61B 5/00(2006.01); A61B 5/02(2006.01); A61B 5/024(2006.01); A61B 5/0245(2006.01); A61B 5/0452(2006.01); A61B 5/11(2006.01); A61B 5/1455(2006.01); G06F 9/451(2018.01); G16H 40/63(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 센서 (sensor), 심장 (heart), 부정맥 (arrhythmia), 시간 (time), 품질 (quality)

### C. DOCUMENTS CONSIDERED TO BE RELEVANT

| Category* | Citation of document, with indication, where appropriate, of the relevant passages | Relevant to claim No. |
|---|---|---|
| Y | KR 10-2019-0050693 A (SAMSUNG ELECTRONICS CO., LTD.) 13 May 2019 (2019-05-13) See paragraphs [0023] and [0030]. | 1-5,7-12,14 |
| A | | 6,13 |
| Y | JP 2017-042386 A (SEIKO EPSON CORP.) 02 March 2017 (2017-03-02) See paragraphs [0067]-[0098], [0133] and [0136]; and figures 4-7, 14 and 15. | 1-5,7-12,14 |
| Y | JP 2020-513876 A (RHYTHM DIAGNOSTIC SYSTEMS, INC.) 21 May 2020 (2020-05-21) See paragraphs [0074], [0096], [0100], [0107] and [0129]. | 2,3,9,10,14 |
| A | KR 10-2020-0111265 A (APPLE INC.) 28 September 2020 (2020-09-28) See entire document. | 1-14 |
| A | KR 10-2019-0121237 A (SAMSUNG ELECTRONICS CO., LTD.) 25 October 2019 (2019-10-25) See entire document. | 1-14 |

☐ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **10 January 2022** | **10 January 2022** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
**Information on patent family members**

International application No.

**PCT/KR2021/013536**

| Patent document cited in search report | | | Publication date (day/month/year) | Patent family member(s) | | | Publication date (day/month/year) |
|---|---|---|---|---|---|---|---|
| KR | 10-2019-0050693 | A | 13 May 2019 | CN | 109745041 | A | 14 May 2019 |
| | | | | DE | 102018117484 | A1 | 09 May 2019 |
| | | | | JP | 2019-084343 | A | 06 June 2019 |
| | | | | TW | 201918222 | A | 16 May 2019 |
| | | | | US | 11147463 | B2 | 19 October 2021 |
| | | | | US | 2019-0133468 | A1 | 09 May 2019 |
| JP | 2017-042386 | A | 02 March 2017 | None | | | |
| JP | 2020-513876 | A | 21 May 2020 | CA | 2886858 | A1 | 10 April 2014 |
| | | | | CA | 2935598 | A1 | 30 July 2015 |
| | | | | CA | 2983771 | A1 | 29 December 2016 |
| | | | | CA | 3045025 | A1 | 21 June 2018 |
| | | | | CN | 104812296 | A | 29 July 2015 |
| | | | | CN | 104812296 | B | 01 May 2018 |
| | | | | CN | 105960197 | A | 21 September 2016 |
| | | | | CN | 107735025 | A | 23 February 2018 |
| | | | | CN | 110087543 | A | 02 August 2019 |
| | | | | EP | 2903509 | A1 | 12 August 2015 |
| | | | | EP | 2903509 | A4 | 01 June 2016 |
| | | | | EP | 2903509 | B1 | 04 September 2019 |
| | | | | EP | 3099224 | A1 | 07 December 2016 |
| | | | | EP | 3099224 | A4 | 27 September 2017 |
| | | | | EP | 3099224 | B1 | 20 May 2020 |
| | | | | EP | 3313287 | A1 | 02 May 2018 |
| | | | | EP | 3313287 | A4 | 20 February 2019 |
| | | | | EP | 3554366 | A1 | 23 October 2019 |
| | | | | EP | 3636148 | A2 | 15 April 2020 |
| | | | | EP | 3636148 | A3 | 20 May 2020 |
| | | | | EP | 3769669 | A1 | 27 January 2021 |
| | | | | ES | 2824126 | T3 | 11 May 2021 |
| | | | | FR | 3015100 | A1 | 19 June 2015 |
| | | | | FR | 3015100 | B1 | 25 December 2015 |
| | | | | JP | 2015-530225 | A | 15 October 2015 |
| | | | | JP | 2017-510390 | A | 13 April 2017 |
| | | | | JP | 2018-126518 | A | 16 August 2018 |
| | | | | JP | 2018-518323 | A | 12 July 2018 |
| | | | | JP | 2019-141704 | A | 29 August 2019 |
| | | | | JP | 2021-053464 | A | 08 April 2021 |
| | | | | JP | 6298063 | B2 | 20 March 2018 |
| | | | | JP | 6539827 | B2 | 10 July 2019 |
| | | | | JP | 6625682 | B2 | 25 December 2019 |
| | | | | JP | 6826735 | B2 | 10 February 2021 |
| | | | | US | 10080527 | B2 | 25 September 2018 |
| | | | | US | 10244949 | B2 | 02 April 2019 |
| | | | | US | 10413251 | B2 | 17 September 2019 |
| | | | | US | 10610159 | B2 | 07 April 2020 |
| | | | | US | 10842391 | B2 | 24 November 2020 |
| | | | | US | 10863947 | B2 | 15 December 2020 |
| | | | | US | 10959678 | B2 | 30 March 2021 |
| | | | | US | 10980486 | B2 | 20 April 2021 |
| | | | | US | 10993671 | B2 | 04 May 2021 |

Form PCT/ISA/210 (patent family annex) (July 2019)

### INTERNATIONAL SEARCH REPORT
#### Information on patent family members

| | International application No. |
|---|---|
| | **PCT/KR2021/013536** |

| Patent document cited in search report | | | Publication date (day/month/year) | Patent family member(s) | | | Publication date (day/month/year) |
|---|---|---|---|---|---|---|---|
| | | | | US | 2014-0100432 | A1 | 10 April 2014 |
| | | | | US | 2014-0189881 | A1 | 03 July 2014 |
| | | | | US | 2015-0094552 | A1 | 02 April 2015 |
| | | | | US | 2015-0096050 | A1 | 02 April 2015 |
| | | | | US | 2015-0148622 | A1 | 28 May 2015 |
| | | | | US | 2015-0148637 | A1 | 28 May 2015 |
| | | | | US | 2015-0148691 | A1 | 28 May 2015 |
| | | | | US | 2015-0170527 | A1 | 18 June 2015 |
| | | | | US | 2016-0302674 | A1 | 20 October 2016 |
| | | | | US | 2018-0028122 | A1 | 01 February 2018 |
| | | | | US | 2018-0177459 | A1 | 28 June 2018 |
| | | | | US | 2019-0029599 | A1 | 31 January 2019 |
| | | | | US | 2019-0320914 | A1 | 24 October 2019 |
| | | | | US | 2020-0008749 | A1 | 09 January 2020 |
| | | | | US | 2020-0229767 | A1 | 23 July 2020 |
| | | | | US | 2021-0100514 | A1 | 08 April 2021 |
| | | | | US | 2021-0145293 | A1 | 20 May 2021 |
| | | | | US | 8935800 | B2 | 13 January 2015 |
| | | | | US | 9582434 | B2 | 28 February 2017 |
| | | | | US | 9773423 | B2 | 26 September 2017 |
| | | | | US | 9782132 | B2 | 10 October 2017 |
| | | | | WO | 2014-055994 | A1 | 10 April 2014 |
| | | | | WO | 2015-113054 | A1 | 30 July 2015 |
| | | | | WO | 2016-210334 | A1 | 29 December 2016 |
| | | | | WO | 2018-112401 | A1 | 21 June 2018 |
| KR | 10-2020-0111265 | A | 28 September 2020 | AU | 2019-100222 | A4 | 11 April 2019 |
| | | | | AU | 2019-100222 | B4 | 12 September 2019 |
| | | | | AU | 2019-234289 | A1 | 01 October 2020 |
| | | | | AU | 2020-230340 | A1 | 01 October 2020 |
| | | | | AU | 2021-261861 | A1 | 02 December 2021 |
| | | | | CN | 110265131 | A | 20 September 2019 |
| | | | | CN | 110729048 | A | 24 January 2020 |
| | | | | CN | 110729048 | B | 13 November 2020 |
| | | | | CN | 111920380 | A | 13 November 2020 |
| | | | | CN | 111920381 | A | 13 November 2020 |
| | | | | CN | 112269617 | A | 26 January 2021 |
| | | | | DK | 179980 | B1 | 27 November 2019 |
| | | | | DK | 180241 | B1 | 08 September 2020 |
| | | | | DK | 180246 | B1 | 11 September 2020 |
| | | | | DK | 201870599 | A1 | 16 October 2019 |
| | | | | DK | 201870600 | A1 | 09 October 2019 |
| | | | | DK | 201870601 | A1 | 16 October 2019 |
| | | | | DK | 201870602 | A1 | 16 October 2019 |
| | | | | DK | 202070395 | A1 | 01 July 2020 |
| | | | | EP | 3596734 | A1 | 22 January 2020 |
| | | | | EP | 3734611 | A1 | 04 November 2020 |
| | | | | EP | 3734612 | A1 | 04 November 2020 |
| | | | | JP | 2021-093127 | A | 17 June 2021 |
| | | | | JP | 2021-512429 | A | 13 May 2021 |
| | | | | JP | 6930038 | B2 | 01 September 2021 |

Form PCT/ISA/210 (patent family annex) (July 2019)

**INTERNATIONAL SEARCH REPORT**
**Information on patent family members**

International application No.

**PCT/KR2021/013536**

| Patent document cited in search report | | | Publication date (day/month/year) | Patent family member(s) | | | Publication date (day/month/year) |
|---|---|---|---|---|---|---|---|
| | | | | JP | 6961057 | B2 | 05 November 2021 |
| | | | | KR | 10-2020-0111266 | A | 28 September 2020 |
| | | | | KR | 10-2021-0084707 | A | 07 July 2021 |
| | | | | KR | 10-2272236 | B1 | 01 July 2021 |
| | | | | US | 10568533 | B2 | 25 February 2020 |
| | | | | US | 10624550 | B2 | 21 April 2020 |
| | | | | US | 11039778 | B2 | 22 June 2021 |
| | | | | US | 11202598 | B2 | 21 December 2021 |
| | | | | US | 2019-0274562 | A1 | 12 September 2019 |
| | | | | US | 2019-0274563 | A1 | 12 September 2019 |
| | | | | US | 2019-0274564 | A1 | 12 September 2019 |
| | | | | US | 2019-0274565 | A1 | 12 September 2019 |
| | | | | US | 2021-0113137 | A1 | 22 April 2021 |
| | | | | WO | 2019-177769 | A1 | 19 September 2019 |
| | | | | WO | 2019-177769 | A4 | 19 September 2019 |
| KR | 10-2019-0121237 | A | 25 October 2019 | CN | 110384479 | A | 29 October 2019 |
| | | | | US | 10849531 | B2 | 01 December 2020 |
| | | | | US | 2019-0313947 | A1 | 17 October 2019 |

Form PCT/ISA/210 (patent family annex) (July 2019)